# EUROPEAN PATENT APPLICATION

(11) **EP 3 981 331 A1**
(43) Date of publication of application: **13.04.2022**
(21) Application number: 21201210.8
(22) Date of filing: 06.10.2021
(51) Int. Cl.: A61B 5/30, A61B 5/308, A61B 5/31, A61B 5/315, A61B 5/332

(54) **COUPLING ARRANGEMENT, ELECTRODE SYSTEM AND BIO-SIGNAL PROCESSING DEVICE FOR BIO-SIGNAL MEASUREMENT AND BIO-SIGNAL MEASUREMENT SYSTEM**

(30) Priority: 07.10.2020 US 202017065028
(71) Applicant: Bittium Biosignals Oy, 70800 Kuopio (FI)
(72) Inventor: Myllykangas, Juha, 70800 Kuopio (FI); Nikula, Arto, 70800 Kuopio (FI)
(74) Representative: Kolster Oy Ab

(57) **Abstract**

A coupling arrangement for a bio-signal measurement, comprises at least two spring connectors (108, 108', 108") of a bio-signal processing device (106), and a holder (104) comprising a combination of a wall (1040) and a substrate, (1020) which form a pocket inside the holder (104), the wall (1040) following an outer contour of the bio-signal processing device (106). The holder has an aperture for inserting the bio-signal processing device (106) into the pocket (104') and removing the bio-signal processing device (106) from the pocket (104'). The holder (104) includes electric conductor lines (102, 102', 102"), which are in a wired electric contact with electrodes of the substrate, the electrodes (112, 112', 112") receiving at least one bio-signal. The electric conductor lines (102, 102', 102") are attached on the substrate inside the pocket (104'), the electric conductor lines (102, 102', 102") and the at least two spring connectors (108, 108', 108") connecting electrically with each other in response to insertion of the bio-signal processing device (106) in the pocket (104').

## Description

### Field

The invention relates to a coupling arrangement for a bio-signal measurement, an electrode system and a bio-signal processing device for bio-signal measurement, and a bio-signal measurement system.

### Background

Because an individual ECG, EEG and/or EOG (ElectroCardioGraphy, ElectroEncephaloGraphy and/or ElectroOculography) patch electrode structure is disposable, it should be as simple as possible. A typical solution has at least some electromechanical way of connecting and fixing the non-disposable measurement device with the disposable single-use electrode structure part.

The measurement devices can be connected with a disposable electrode structure using assembled connectors such as snap connectors, a USB-connector etc. Some devices use PoGo-pins.

The electromechanical connectors are often structurally and functionally complicated and expensive parts to both manufacture and assemble on the disposable patch electrode. The electrode structure with the electromechanical connectors should, thus, further be developed and simplified.

### Brief description

The present invention seeks to provide an improvement to the electromechanical connections.

The invention is defined by the independent claims. Embodiments are defined in the dependent claims.

### List of drawings

Example embodiments of the present invention are described below, by way of example only, with reference to the accompanying drawings, in which
Figure 1A to 1C illustrate an example of a bio-signal measurement system;
Figure 2 illustrates a commercial spring contact component;
Figures 3A and 3B illustrate an example of a projection and a recess for a klick-coupling;
Figure 4 illustrates an example of a via;
Figure 5 illustrates an example of a bio-signal processing device seen from above;
Figure 6 illustrates of an example of the bio-signal processing device as a side view; and
Figure 7 illustrates a flow chart of an example of a connection method for a coupling arrangement of a bio-signal measurement.

### Description of embodiments

The following embodiments are only examples. Although the specification may refer to "an" embodiment in several locations, this does not necessarily mean that each such reference is to the same embodiment(s), or that the feature only applies to a single embodiment. Single features of different embodiments may also be combined to provide other embodiments. Furthermore, words "comprising" and "including" should be understood as not limiting the described embodiments to consist of only those features that have been mentioned and such embodiments may also contain features/structures that have not been specifically mentioned. All combinations of the embodiments are considered possible if their combination does not lead to structural or logical contradiction.

It should be noted that while Figures illustrate various embodiments, they are simplified diagrams that only show some structures and/or functional entities. The connections shown in the Figures may refer to logical or physical connections. It is apparent to a person skilled in the art that the described apparatus may also comprise other functions and structures than those described in Figures and text. It should be appreciated that details of some functions, structures, and the signalling used for measurement and/or controlling are irrelevant to the actual invention. Therefore, they need not be discussed in more detail here.

An idea in this document is to handle the electromechanical connection from a bio-signal processing device 106 to disposable electrode structure (substrate 1020 with least two electric conductor lines 102, 102', 102" and electrodes 112, 112', 112") with spring connectors 108, 108', 108", the spring connectors 108, 108', 108" being required only on a side of the bio-signal processing device 106. The spring connectors 108, 108', 108" may be industry standard springs. The mechanical holding force of the interconnection is then handled with separate, dummy mechanical plastic parts i.e. a holder 104. The holder 104 holds the bio-signal processing device 106 and electrode structure together.

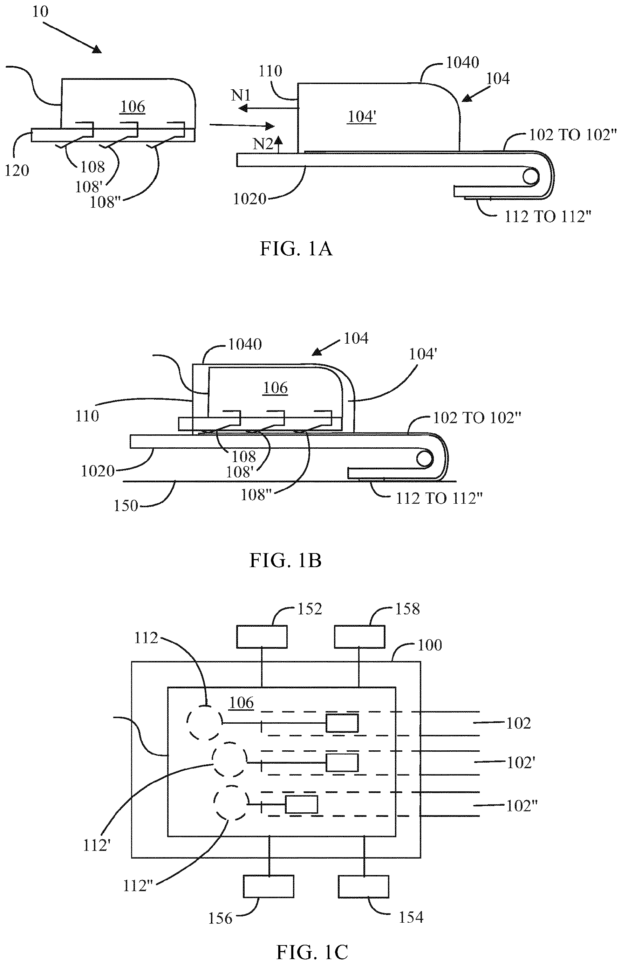

Figure 1A to 1C illustrate an example of a bio-signal measurement system 10. Examples of Figures 1A and 1B show the bio-signal measurement system 10 as a side view. The example of Figure 1C shows the bio-signal measurement system 10 from a top.

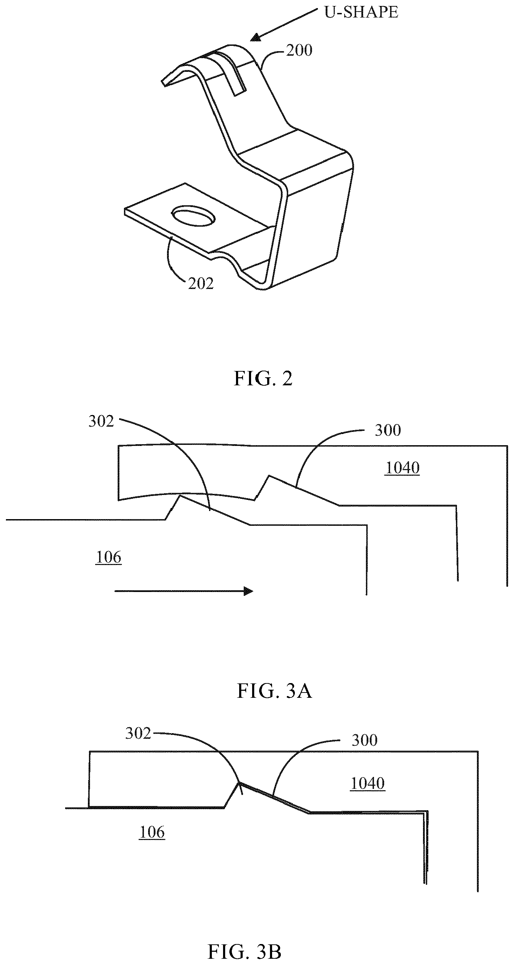

The bio-signal measurement system 10 comprises a bio-signal processing device 106, which has at least two spring connectors 108, 108', 108" (see also Figure 2), a substrate 1020, which has at least two electric conductor lines 102, 102', 102", and a holder 104.

The bio-signal processing device 106 may be an electronic device, which may convert an analog bio-signal it receives to a digital bio-signal. The bio-signal processing device 106 may also filter the bio-signal in an analog or in a digital form. Additionally or alternatively, the bio-signal processing device 106 may perform data processing of the bio-signal, and it may also store data of the bio-signal and/or a result of its processing. The bio-signal may be related to heart rate variability, electrocardiogram, electromyogram, electroencephalogram or the like for example. The bio-signal processing device 106 may be a measurement device that performs the bio-signal measurement. However, the bio-signal processing device 106 may also be connected to another electronic device such as a computer or the like during the measurement or after the measurement.

The substrate 1020 may be made of polymer or cloth, for example. The polymer may be made of plastic, for example. In an embodiment, the polymer may be made of PET (PolyEthylene Terephthalate) and/or PU (PolyUretan). In an embodiment, the polymer may be made of material based on wood fibers, which in an embodiment may be recycled which makes it environmental friendly. The substrate 1020 with the least two electric conductor lines 102, 102', 102" may be made of material which is harmless to environment and which may be burnt because the substrate 1020 with the least two electric conductor lines 102, 102', 102" is disposable. In an embodiment, the least two electric conductor lines 102, 102', 102" may be made of electrically conductive material based on carbon. In an embodiment, the least two electric conductor lines 102, 102', 102" may be made of silver chloride (AgCl), for example.

The holder 104 comprises a combination of a wall 1040, which follows an outer contour of the bio-signal processing device 106 and is fitted to it, and the substrate 1020. The wall 1040 may be made of polymer such as plastic, for example. The polymer structure of the wall 1040 may be stretching and flexible. The wall 1040 and the substrate 1020 form a pocket 104' inside the holder 104. The pocket 104' is a cavity or a tube that is fit to the bio-signal processing device 106. The wall 1040 may have a fully continuous structure, it may have holes regularly or irregularly, or it may have a net structure. In an example of Figure 1A, the bio-signal processing device 106 is being moved toward the holder 104. In an example of Figure 1B, the bio-signal processing device 106 is inside the holder 104. The holder 104 may be molded, for example.

The pocket 104' has an aperture 110 for inserting the bio-signal processing device 106 into the pocket 104' and removing the bio-signal processing device 106 from the pocket 104'. That is, the pocket 104' may have only one aperture 110. A width and a height of the aperture 110 may be about the same as a width and a thickness of the bio-signal processing device 106. In an embodiment, a maximum width and a maximum height of the aperture 110 may be about the same as a maximum width and a maximum thickness of the bio-signal processing device 106. In an embodiment, the thickness and the width of the casing of the bio-signal processing device 106 may be at least approximately constant for its full length.

The substrate 1020 has the at least two electric conductor lines 102, 102', 102", which are in a wired electric contact with electrodes 112, 112', 112" of the substrate 1020 and receive at least one bio-signal from skin 150, inside the pocket 104'. The electrodes 112, 112', 112" are configured to be attached to the skin 150 of a mammal that may be a human being, a cat, a dog, a horse, a cow, a sheep, a pig or the like for example.

The electric conductor lines 102, 102', 102" and the at least two spring connectors 108, 108', 108" connect electrically with each other in response to insertion of the bio-signal processing device 106 into the pocket 104'.

In this manner, the disposable part i.e. the substrate 1020 with the least two electric conductor lines 102, 102', 102" and the electrodes 112, 112', 112" has a minimized amount of material and electronic and mechanical components. It is simple to manufacture, i.e. saves material and manufacturing cost, and simple to dispose, which both can be done harmlessly to people and environment.

The pocket 104' is a free space or volume into which at least a part of the bio-signal processing device 106 may fit accurately. A degree of precision with which the surfaces of the bio-signal processing device 106 and the wall 1040 are adapted to each other may be high enough to enable operation with one hand when inserting the bio-signal processing device 106 into or removing the bio-signal processing device 106 from the holder 104. Friction between an outer surface of the bio-signal processing device 106 and an inner surface of the holder 104 may keep the bio-signal processing device 106 in the holder 104 even under accelerations caused by sport activities or in upside down positions. The fit between the bio-signal processing device 106 and the holder 104 may be rather tight resulting in a suitable friction and suction force. The pocket 104' may have the wall 1040 round the bio-signal processing device 106 in a continuous hemispherical manner, which is more secure and efficient than a wall that has a shape of a band round the bio-signal processing device 106, for example. Still, the hemispherical wall 1040 allows easy removal of the bio-signal processing device 106 from the pocket 104'.

Figure 2 illustrates an example of a commercial spring contact component that can be used as the spring connectors 108, 108', 108". A spring part 200 may bend under pressure while a support and attachment part 202 can be attached to the bio-signal processing device 106. The spring connectors 108, 108', 108" may be attached to a printed board or other support 120 using surface mounting. In order to improve positioning in an embodiment, the spring connectors 108, 108', 108" may have at least one positioning extension such as a spike, for example, that could go into or through the printed board or other support 120. Alternatively or additionally with respect to the at least one spike, edges of the spring connectors 108, 108', 108" may be bend about 90° or between about 45° and about 135°, for example, downward. Still alternatively or additionally, the surface area that contacts the least two electric conductor lines 102, 102', 102" may be made larger by a suitable bending or shape of the spring connectors 108, 108', 108" in order to decrease a pressure between the spring connectors 108, 108', 108" and the least two electric conductor lines 102, 102', 102". In an embodiment, the surface area of the spring connectors 108, 108', 108" that contacts the least two electric conductor lines 102, 102', 102" is curved to have at least approximately a shape of the letter U and the contact is formed by the convex side the shape for making the spring connectors 108, 108', 108" easy to move over the least two electric conductor lines 102, 102', 102" while maintaining continuous contact with the least two electric conductor lines 102, 102', 102". In an embodiment, the spring connectors 108, 108', 108" may be side by side. In an embodiment, at least two spring connectors 108, 108', 108" may be one after another.

In an embodiment, the bio-signal processing device 106 may comprise or be coupled with at least one of the following: an oxygen saturation sensor 152, a galvanic skin resistance sensor 154, a magnetic sensor 156, and an ultrasound sensor 158.

In an embodiment, the tissue oxygen saturation sensor 152 may be based on infrared spectroscopy. A person skilled in the art is familiar with the oxygen saturation sensors, *per se,* which is why the oxygen saturation sensors do not need to be described in detail here. Because the bio-signal processing device 106 is adjacent or directly adjacent to the skin 150 of the mammal, the oxygen saturation sensor 152 may also be in physical contact with the bio-signal processing device 106 or the oxygen saturation sensor and the oxygen saturation sensor may have only a short wire therebetween. The bio-signal processing device 106 may receive electric signals from the oxygen saturation sensor 152 and determine the oxygen saturation of the blood, for example.

In an embodiment, the galvanic skin resistance sensor 154 may be measure conductivity of the skin 150, which varies with an amount and quality of sweat on the skin 150. The bio-signal processing device 106 may receive electric signals from the galvanic skin resistance sensor 154 and determine condition of liver and/or kidneys, for example, because they affect the quality of the sweat.

In an embodiment, the magnetic sensor 156 may be used to measure a flow of blood in vessels. The bio-signal processing device 106 may receive electric signals from the magnetic sensor 156 and determine the flow.

In an embodiment, the ultrasound sensor 158 may transmit and receive ultrasound signals to and from tissue. The bio-signal processing device 106 may receive electric signals from the ultrasound sensor 158 and form at least one image of tissue of the mammal and/or measure a flow of blood in vessels, for example.

As can be seen from Figures 1A to 1C, an electrode system for the bio-signal measurement comprises the holder 104 comprising a combination of the wall 1040 and the substrate 1020, which are configured to form the pocket 104'. The wall 1020 is fitted to an outer contour of a bio-signal processing device 106.

The holder 104 has the aperture 110 for inserting the bio-signal processing device 106 into the pocket 104' and removing the bio-signal processing device 106 from the pocket 104'.

The holder 104 includes the at least two electric conductor lines 102, 102', 102", which are in a wired electric contact with the electrodes 112, 112', 112" of the substrate 1020, and the electrodes 112, 112', 112" are configured to receive the at least one bio-signal.

The electric conductor lines 102, 102', 102" are attached on the substrate 1020 inside the pocket 104', and the electric conductor lines 102, 102', 102" are configured to electrically connect and contact with the at least two spring connectors 108, 108', 108" of the bio-signal processing device 106 in response to insertion of the bio-signal processing device 106 into the pocket 104'.

In an embodiment, a normal N1 of the aperture 110 may be perpendicular to a normal N2 of the substrate 1020 at the aperture 110.

In an embodiment, the wall 1040 is made of polymer. The polymer may be plastic or resin, for example.

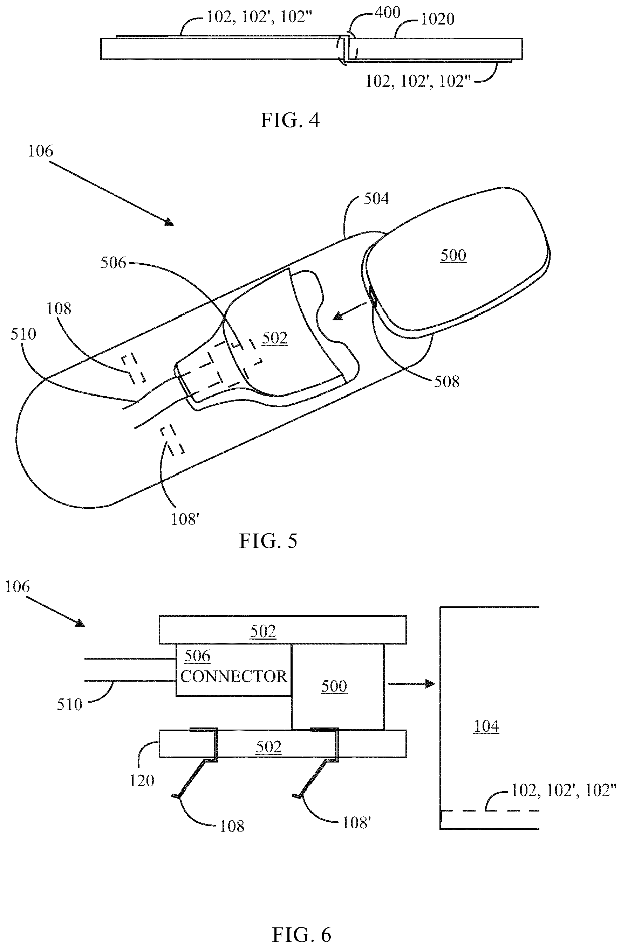

In an embodiment examples of which are shown in Figures 1A and 1B, the substrate 1020 may be folded back on itself for allowing the electric conductor lines 102, 102', 102" to be made only on one side. In an embodiment an example of which is shown in Figure 4, the substrate 1020 may have the electric conductor lines 102, 102', 102" on both sides and the substrate 1020 may have vias 400 for electrically contacting the electric conductor lines 102, 102', 102" on both sides.

It can also be seen that the bio-signal measurement system 10 has a coupling arrangement for the bio-signal measurement. The coupling arrangement comprises at least two spring connectors 108, 108', 108" of a bio-signal processing device 106, and a holder 104 comprising a combination of a wall 1040 and a substrate 1020, which are configured to form a pocket 104' inside the holder 104. The wall 1040 of the pocket 104' is configured to follow an outer contour of the bio-signal processing device 106.

The holder 104 has the aperture 110 for inserting the bio-signal processing device 106 into the pocket 104' and removing the bio-signal processing device 106 from the pocket 104'.

The holder 104 includes at least two electric conductor lines 102, 102', 102", which are in a wired electric contact with electrodes 112, 112', 112" of the substrate 1020, and the electrodes 112, 112', 112" are configured to receive at least one bio-signal.

The electric conductor lines 102, 102', 102" are attached on the substrate 1020 inside the pocket 104', and the electric conductor lines 102, 102', 102" and the at least two spring connectors 108, 108', 108" are configured to electrically connect with each other in response to insertion of the bio-signal processing device 106 in the pocket 104'.

In an embodiment, the spring connectors 108, 108', 108" may slide on the electric conductor lines 102, 102', 102" in response to an inserting movement of the bio-signal processing device 106 in the pocket 104'. The electric conductor lines 102, 102', 102" may be straight like the pocket 104' in the direction of the electric conductor lines 102, 102', 102" or may curve in a corresponding manner to the electric conductor lines 102, 102', 102". The sliding movement cleanses contact areas of the electric conductor lines 102, 102', 102" and the spring connectors 108, 108', 108", which improves the electric contact therebetween.

In an embodiment, the spring connectors 108, 108', 108" and the electric conductor lines 102, 102', 102" are pressed against each other by a spring force of the spring connectors 108, 108', 108" in response to a pressure of the wall 1040 against the bio-signal processing device 106 resulting from the insertion of the bio-signal processing device 106 into the pocket 104' and presence of the bio-signal processing device 106 in the pocket 104'.

In an embodiment, the spring connectors 108, 108', 108" may bend under pressure of the wall 1040 against the bio-signal processing device 106 resulting from the insertion of the bio-signal processing device 106 into the pocket 104' and the presence of the bio-signal processing device 106 in the pocket 104'.

Figures 3A illustrates an example where the bio-signal processing device 106 is inserted into the pocket 104'. In an embodiment, the wall 1040 may have a recess 300 and the bio-signal processing device 106 may have a projection 302 as a counterpart to the recess 300. The projection 302 may stretch the wall 1040 a little because of the fit. The projection 302 may fall in the recess 300 in response to a full and correct insertion of the bio-signal processing device 106 into the pocket 104' as shown in Figure 3B. Alternatively, the wall 1040 may have a projection and the bio-signal processing device 106 may have a recess as a counterpart to the projection (not shown in Figures because it is a simple variation of the embodiment illustrated in Figures 3A and 3B). A user may hear a "klick" sound and feel it when it happens such that the user knows that the connection is proper.

Figure 5 illustrates an example of the bio-signal processing device 106 seen from above. Thus, the bio-signal device 106 may comprise a bio-signal processing unit 500, which performs data processing and is to be inserted in a connection chamber 502 (see arrow), the connection chamber 502 for the bio-signal processing unit 500, and a support structure 504, which carries the connection chamber 502. The connection chamber 502 has a device connector 506 inside the connection chamber 502, and the device connector 506 is configured to be connected to a counter connector 508 of the bio-signal processing unit 500. The device connector 506 and the counter connector 508 may be a pair of USB-connectors which are connectable to each other. That is the device connector 506 may be a male USB-connector and the counter connector 508 may be a female USB-connector or vice versa. The device connector 506 may be pigtailed, see wire 510, in order to connect the bio-signal processing unit 500 with an external device such as a wireless transceiver or a computer. Alternatively, the wire 510 may be coupled with the at least two spring connectors 108, 108' for transferring electric signals from the electrodes 112 to 112" through the at least two spring connectors 108, 108' to the bio-signal processing unit 500. The support structure 504 may comprise a printed circuit board, for example.

In an embodiment an example of which is illustrated in Figure 6. Figure 6 shows the bio-signal processing device 106 of Figure 5 and the holder 104 seen from a side. The at least two spring connectors 108, 108' penetrate and are fixed to a wall 504 of the connection chamber 502. The separate parts of the bio-signal processing device 106 may be fixed to each other using an adhesive that can be hardened in ultraviolet light, for example.

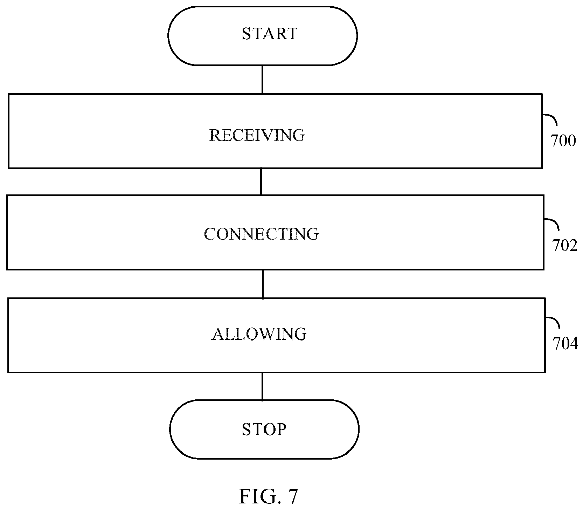

Figure 7 illustrates a flow chart of an example of a connection method for a coupling arrangement of a bio-signal measurement. In step 700, a bio-signal processing device 106 is received through an aperture 110 of a holder 104, which comprises a combination of a wall 1040 and a substrate 1020 that form a pocket 104' inside the holder 104, into the pocket 104', the wall 1040 following an outer contour of the bio-signal processing device 106. In step 702, electrically electric conductor lines 102, 102', 102", which are attached on the substrate 1020 inside the pocket 104' and have a wired electric contact with electrodes 112, 112', 112" of the substrate 1020 for receiving at least one bio-signal, and at least two spring connectors 108, 108', 108" are connected with each other in response to insertion of the bio-signal processing device 106 in the pocket 104'. In step 704, a removal of the bio-signal processing device 106 from the pocket 104' is allowed and enabled through the aperture 110.

This solution can be used to minimize the needed materials and electronics on the disposable part, i.e. the combination of the wall 1040 and the substrate 1020 with the electric conductor lines 102, 102', 102', and therefore reduce price, manufacturing time, environmental burden etc.

The bio-signal measurement system can be made to fulfil IP67-code which means the bio-signal measurement system is water (1 m deep) and dust resistant. Namely, water in the pocket 104' can easily be removed because water clears out of the pocket through the aperture 110 and the remaining water easily dries such that long-term shortcuts after a shower can be avoided.

The solution described above introduces a cheap solution & device concept to form the needed connection between the bio-signal device 106 and the combination of the wall 1040 and the substrate 1020 with the electric conductor lines 102, 102', 102". This type of connection could be used in many different kind and type of bio-signal measurements such as ECG, EEG and EOG.

Some of key points of the solution described above:
1. The use of conventional RF shielding slide spring contacts to contact directly on the electrode printed AgCl-conductor surface ― no need for assembled mating parts on the disposable part.
2. The use of slide spring contact is beneficial on this kind of body attached product because the mating area is large compared to PoGo-pins, for example, and the slide-action cleans the connection surfaces every time it is used. Large even surfaces also reduce a risk of motion artefacts due to poor connection.
3. A unique Mechanical "Slide and Click" ― structure together with Device/Adapter and Electrode Holder.
4. A unique Device/Adapter spring contact arrangement to avoid moisture pockets.
5. A unique Electrode Holder -structure to avoid moisture pockets and enable easy slide of the bio-signal processing device 106.
6. A unique conductive layer construction where conductive layer tail may be flipped 180° on top of the electrode to meet the Slide Spring Contacts and therefore the need for 2-layer conductive layer construction with vias may be avoided.
7. Minimized overall layer structure of the Patch Electrode.
It will be obvious to a person skilled in the art that, as technology advances, the inventive concept can be implemented in various ways. The invention and its embodiments are not limited to the example embodiments described above but may vary within the scope of the claims.

## Claims

1. A coupling arrangement for a bio-signal measurement, **characterized in that** the coupling arrangement comprises at least two spring connectors (108, 108', 108") of a bio-signal processing device (106), and a holder (104) comprising a combination of a wall (1040) and a substrate (1020), which are configured to form a pocket (104') inside the holder (104), the wall (1040) being configured to follow an outer contour of the bio-signal processing device (106);
the holder (104) has an aperture (110) for inserting the bio-signal processing device (106) into the pocket (104') and removing the bio-signal processing device (106) from the pocket (104');
the holder (104) includes at least two electric conductor lines (102, 102', 102"), which are configured to be in a wired electric contact with electrodes (112, 112', 112") of the substrate (1020), the electrodes (112, 112', 112") being configured to receive at least one bio-signal; and
the electric conductor lines (102, 102', 102") are attached on the substrate (1020) inside the pocket (104'), the electric conductor lines (102, 102', 102") and the at least two spring connectors (108, 108', 108") are configured to electrically connect with each other in response to insertion of the bio-signal processing device (106) in the pocket (104').

2. The coupling arrangement of claim 1, **characterized in that** the spring connectors (108, 108', 108") are configured to slide on the electric conductor lines (102, 102', 102") in response to an inserting movement of the bio-signal processing device (106) in the pocket (104').

3. The coupling arrangement of claim 1, **characterized in that** the spring connectors (108, 108', 108") and the electric conductor lines (102, 102', 102") are configured to be pressed against each other by a spring force of the spring connectors (108, 108', 108") in response to a pressure of the wall (1040) against the bio-signal processing device (106) resulting from insertion of the bio-signal processing device (106) into the pocket (104') and presence of the bio-signal processing device (106) in the pocket (104').

4. The coupling arrangement of claim 1, **characterized in that** the spring connectors (108, 108', 108") are configured to bend under pressure of the wall (1040) against the bio-signal processing device (106) resulting from insertion of the bio-signal processing device (106) into the pocket (104') and presence of the bio-signal processing device (106) in the pocket (104').

5. The coupling arrangement of claim 1, **characterized in that** the wall (1040) has a projection and the bio-signal processing device (106) has a recess as a counterpart to the projection, or the wall (1040) has a recess (300) and the bio-signal processing device (106) has a projection (302) a counterpart to the recess (300); and the projection (302) being configured to fall in the recess (300) in response to a full and correct insertion of the bio-signal processing device (106) into the pocket (104').

6. The coupling arrangement of claim 1, **characterized in that** a normal (N1) of the aperture (110) is perpendicular to a normal (N2) of the substrate (1020) at the aperture (110).

7. The coupling arrangement of claim 1, **characterized in that** the wall (1040) is made of polymer.

8. An electrode system for a bio-signal measurement, **characterized in that** the electrode system comprises a holder (104) comprising a combination of a wall (1040) and a substrate (1020), which are configured to form a pocket (104'), and the wall (1040) being fitted to an outer contour of a bio-signal processing device (106);
the holder (104) has an aperture (110) for inserting the bio-signal processing device (106) into the pocket (104') and removing the bio-signal processing device (106) from the pocket (104');
the holder (104) includes at least two electric conductor lines (102, 102', 102"), which are configured to be in a wired electric contact with electrodes (112, 112', 112") of the substrate (1020), the electrodes (112, 112', 112") being configured to receive at least one bio-signal; and
the electric conductor lines (102, 102', 102") are attached on the substrate (1020) inside the pocket (104'), and the electric conductor lines (102, 102', 102") are configured to electrically connect the at least two spring connectors (108, 108', 108") of the bio-signal processing device (106) in response to insertion of the bio-signal processing device (106) into the pocket (104').

9. The electrode system of claim 8, **characterized in that** a normal (N1) of the aperture (110) is perpendicular to a normal (N2) of the substrate (1020) at the aperture (110).

10. The electrode system of claim 8, **characterized in that** the wall (1040) is made of polymer.

11. The electrode system of claim 8, **characterized in that** the substrate (1020) is folded back on itself for allowing the electric conductor lines (102, 102', 102") to be only on one side.

12. A bio-signal processing device for a bio-signal measurement, **characterized in that** the bio-signal processing device (106) comprises at least two spring connectors (108, 108', 108"), which are configured to electrically connect with electric conductor lines (102, 102', 102") that are attached on a substrate (1020), configured to be in a wired electric contact with electrodes (112, 112', 112") of at least one bio-signal reception and at least partly inside a pocket (104') of the substrate (1020) and a wall (1040) that is configured to follow an outer contour of the bio-signal processing device (106) in order to form a holder (104) for the bio-signal processing device (106), in response to insertion of the bio-signal processing device (106) in the pocket (104') through an aperture (110).

13. A bio-signal measurement system, **characterized in that** the bio-signal measurement system (10) comprises a bio-signal processing device (106), which has at least two spring connectors (108, 108', 108"), a substrate (1020), which has at least two electric conductor lines (102, 102', 102"), and a holder (104);
the holder (104) comprises a combination of a wall (1040), which is configured to follow an outer contour of the bio-signal processing device (106), and the substrate (1020), the wall (1040) and the substrate (1020) are configured to form a pocket (104') inside the holder (104);
the pocket (104') has an aperture (110) for inserting the bio-signal processing device (106) into the pocket (104') and removing the bio-signal processing device (106) from the pocket (104');
the substrate (1020) has the at least two electric conductor lines (102, 102', 102"), which are configured to be in a wired electric contact with electrodes (112, 112', 112") of the substrate (1020) and to receive at least one bio-signal, inside the pocket (104'); and
the electric conductor lines (102, 102', 102") and the at least two spring connectors (108, 108', 108") are configured to electrically connect with each other in response to insertion of the bio-signal processing device (106) into the pocket (104').

14. The bio-signal measurement system of claim 10, **characterized in that** the bio-signal processing device (106) comprises or is coupled with at least one of the following: an oxygen saturation sensor (152), a galvanic skin resistance sensor (154), a magnetic sensor (156), and an ultrasound sensor (156).

15. A connection method, the connection method for a coupling arrangement of a bio-signal measurement, **characterized by** receiving (700), by a holder (104) comprising a combination of a wall (1040) and a substrate (1020) that form a pocket (104') inside the holder (104), a bio-signal processing device (106) through an aperture (110) of the holder (104) into the pocket (104'), the wall (1040) following an outer contour of the bio-signal processing device (106);
connecting (702) electrically electric conductor lines (102, 102', 102"), which are attached on the substrate (1020) inside the pocket (104') and have a wired electric contact with electrodes (112, 112', 112") of the substrate (1020) for receiving at least one bio-signal, and at least two spring connectors (108, 108', 108") with each other in response to insertion of the bio-signal processing device (106) in the pocket (104'); and
allowing (704) a removal of the bio-signal processing device (106) from the pocket (104') through the aperture (110).
